(19) Europäisches Patentamt
European Patent Office
Office européen des brevets

(11) EP 1 322 161 B1

(12) **EUROPÄISCHE PATENTSCHRIFT**

(45) Veröffentlichungstag und Bekanntmachung des Hinweises auf die Patenterteilung:
**16.11.2005 Patentblatt 2005/46**

(21) Anmeldenummer: **01976208.7**

(22) Anmeldetag: **10.09.2001**

(51) Int Cl.[7]: **A01N 43/90**
// (A01N43/90, 53:00, 51:00, 43:90), A01N43:56

(86) Internationale Anmeldenummer:
**PCT/EP2001/010425**

(87) Internationale Veröffentlichungsnummer:
**WO 2002/023991 (28.03.2002 Gazette 2002/12)**

# (54) VERWENDUNG VON RIBOFLAVIN UND FLAVINDERIVATEN ALS INHIBITOREN VON CHITINASEN

USE OF RIBOFLAVIN AND FLAVIN DERIVATIVES AS CHITINASE INHIBITORS

UTILISATION DE RIBOFLAVINE ET DE DERIVES DE FLAVINE EN TANT QU'INHIBITEURS DE CHITINASES

(84) Benannte Vertragsstaaten:
**AT BE CH CY DE DK ES FI FR GB GR IE IT LI LU MC NL PT SE TR**

(30) Priorität: **19.09.2000 DE 10046267**

(43) Veröffentlichungstag der Anmeldung:
**02.07.2003 Patentblatt 2003/27**

(73) Patentinhaber: **Bayer HealthCare AG**
**51368 Leverkusen (DE)**

(72) Erfinder:
- **TUBERG, Andreas**
  **42781 Haan (DE)**
- **GUTSMANN, Volker**
  **42799 Leichlingen (DE)**
- **OMURA, Satosho**
  **Tokyo 157-0013 (JP)**
- **SHIOMI, Kazuro**
  **Tokyo 150-0013 (JP)**

(56) Entgegenhaltungen:
**WO-A-00/04930** **DE-A- 4 139 752**
**FR-A- 2 760 941**

- **DATABASE CA [Online] CHEMICAL ABSTRACTS SERVICE, COLUMBUS, OHIO, US; D.D.TZENG ET AL.: "Biocidal activity of mixtures of methionine ans riboflavine against plant pathogenic fungi and bacteria and possible modes of action" retrieved from STN-INTERNATIONAL, accession no. 111:169241 CA XP002187645 & MYCOLOGIA, Bd. 81, Nr. 3, 1989, Seiten 404-412,**
- **DATABASE CA [Online] CHEMICAL ABSTRACTS SERVICE, COLUMBUS, OHIO, US; S.Y.WANG ET AL.: "Methionine-riboflavin mixtures with surfactants and metal ions reduce powdery mildew infections in strawbwrry plants" retrieved from STN-INTERNATIONAL, accession no. 130:106409 CA XP002187646 & J.AM.SOC.HORTIC.SCI., Bd. 123, Nr. 6, 1998, Seiten 987-991,**
- **DATABASE CA [Online] CHEMICAL ABSTRACTS SERVICE, COLUMBUS, OHIO, US; Z.ZWOLINSKA-SNIATALOWA ET AL.: "Effect of riboflavine and plant phospholipids on Colorado potato beetle (Leptinotarsa decemlineata) physiology" retrieved from STN-INTERNATIONAL, accession no. 75:106842 CA XP002187647 & ROCZ.NAUK.ROLN., SER. E, Bd. 1, Nr. 2, 1971, Seiten 95-112,**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

(Forts. nächste Seite)

- DATABASE CA [Online] CHEMICAL ABSTRACTS SERVICE, COLUMBUS, OHIO, US; A.A.AVER'YANOV ET AL.: "Active oxygen-associated control of rice blast disease by riboflavin and roseoflavin" retrieved from STN-INTERNATIONAL, accession no. 134:111564 CA XP002187648 & BIOCHEMISTRY (MOSCOW), Bd. 65, Nr. 11, 2000, Seiten 1292-1298,
- DATABASE WPI Section Ch, Week 197615 Derwent Publications Ltd., London, GB; Class B03, AN 1976-27237X XP002187649 & JP 51 023299 A (NISSHIN FOODPROD KK), 24. Februar 1976 (1976-02-24)

## Beschreibung

Die Erfindung betrifft die Verwendung von Riboflavin und Flavinderivaten mit Chitinase-inhibitorischer Wirkung zur Bekämpfung von Arthropoden, Nematoden sowie von Chitin enthaltenden Pilzen.

Im Bereich Landwirtschaft stellen Arthropoden als Pflanzenschädlinge, Parasiten und Hygieneschädlinge ein weltweites Problem für Nahrungsmittelproduktion und Gesundheit von Mensch und Tier dar.

Synthetisch chemische Verbindungen sind neben den klassischen Ansätzen aus der Natur erst seit etwa 60 Jahren intensiv im Einsatz. Dennoch entwickeln insbesondere bei Dauereinsatz nahezu alle Schädlinge Resistenzen oder Toleranzen gegenüber den Wirkstoffen. Die Mechanismen der Wirkstoffneutralisierung sind dabei vielfältig und reichen von simplen Verhaltensänderungen, über Aufnahmebarrieren und Metabolismus bis zur Insensitivität des eigentlichen Zielproteins der Substanzen.

Es ist daher von grosser Bedeutung zur Unterstützung bestehender Behandlungskonzepte neue Wirkmechanismen mit neuen Wirkstoffklassen zu finden und die Lebenszeit dieser neuen Stoffe durch Kombination oder Rotation mit bekannten Strukturen zu optimieren.

Es ist allgemein bekannt, dass Arthropoden ein Exoskelett besitzen, wodurch sie sich von den meisten anderen Lebewesen unterscheiden. Ein wichtiger Bestandteil dieser Arthropodencuticula ist Chitin. Es ist ebenfalls bekannt, dass Arthropoden in ihrer Entwicklung von der Larve zum Adulten diese starre Aussenhaut mehrfach wechseln müssen, um wachsen zu können. Dieser Häutung genannte Prozess unterliegt einer komplexen hormonellen Steuerung, bei der verschiedene Proteine gebildet oder reprimiert werden. Ein Teil dieser Proteine ist für den Abbau alter Cuticula und die Cuticula-Neusynthese verantwortlich [K-D. Spindler, In: K. Scheller (ed.) The larval serum proteins of insects. Thieme, Stuttgart: 135-150 (1983)].

So ist der Cuticulabestandteil Chitin, ein β-1-4-N-Acetylglucosamin-Homopolymer in der Endocuticulaschicht Lieferant der für die zur Chitinneusynthese benötigten N-Acetylglucosamin Bausteine. Dazu werden zum Zeitpunkt der Häutung Enzyme von der unter der Cuticula liegenden Epidermis exkretiert, die Chitin verdauen. Diese Chitinasen spalten das Chitinpolymer an zufälligen Stellen bis zu Dimeren des N-Acetylglucosamins = Chitobiose (Endochitinasen 3.2.1.14) und N-Acetylglucosamin. Die Spaltung der Dimere zu N-Acetylglucosamin Monomeren sowie ein Abspalten von N-Acetylglucosamin vom nicht-reduzierenden Ende der Chitinkette (Exochitinasereaktion) wird auch von N-Acetylglucosaminidasen = Hexasaminidasen (3.2.1.50) katalysiert.

Chitin ist aber nicht nur in Arthropoden nachweisbar. Es bildet auch einen wichtigen Bestandteil der pilzlichen Zellwände. Chitin und chitinolytische Aktivität konnte auch in Nematoden nachgewiesen werden. Somit eignen sich Stoffe die in den Chitin-Metabolismus eingreifen nicht zur Bekämpfung von Arthropoden sondern auch zum Einsatz als Fungizide oder Nematizide.

Es wurde in der Literatur schon wiederholt vorgeschlagen den Chitin-Metabolismus als Ansatzpunkt zur Entwicklung neuer und sicherer Pestizide einzusetzen [Spindler, K-D., Spindler-Barth, M. & M. Londershausen, Parasitol. Res. 76:283-288 (1990); E. Cohen, Arch. Insect Biochem. Physiol. 22: 245-261 (1993)]. Mit den Benzoylphenylharnstoffen wurde erstmals eine kommerziell verwertbare Substanzklasse gefunden, die einen Einfluss auf die Chitinbiosynthese hat. Allerdings gibt es bisher keine kommerziell geeigneten Substanzen, die die Chitinsynthase (EC 3.2.1.16) direkt hemmen. Für die Chitinase (EC 3.2.1.14) wurden Inhibitoren beschrieben, die aber bisher aufgrund nicht ausreichender in-vivo Wirksamkeit nicht kommerziell verwendet werden [Sakuda, S., A. Isogai, S. Matsumoto, A. Suzuki & K. Koseki, Tetrahedron Lett. 27, 2475-2478 (1986); Koga, D., A. Isogai, S. Sakuda, S. Matsumoto, A. Suzuki, S. Kimura & A. Ide, Agric. Biol. Chem. 51, 471-476 (1987)].

Ein empfindliches Messprinzip, mit dem spezifisch Endochitinase-Aktivitäten in kleinen Volumina bestimmt werden können, ist bereits im Prinzip bekannt [McCreath, K.J. & G.W. Gooday, J. Microbiol. Meth. 14, 229-237 (1992)].

WO 00/04930 betrifft ein Verfahren und eine Apparatur zur Inaktivierung biologischer Kontomination mit Hilfe von Fotosensibilisatoren, wie zum Beispiel Flavinen.

FR-A-2760941 betrifft ein phytopharmazeutisches Mittel und ein Verfahren zum Schutz von Pflanzen, Keimlingen und Saatgut gegen phytopathogene Pilze.

DE 4139752 betrifft ein Mittel, dessen Herstellung sowie seine Verwendung zur vorbeugenden und/oder nachträglichen Behandlung von Haustieren gegen Zeckenbefall, wobei das Mittel einen Vitamin B aufweisenden Basisstoff enthält.

Z. Zwolinska-Sniatalowa et al. in CA 75:106842 (Rocz. Nauk. Roln., Ser. E, Bd.1, Nr. 2, 1971, Seiten 95-112) beschreibt die Auswirkung von Riboflavin und Pflanzenphospholipiden auf die Physiologie bestimmter Kartoffelkäfer.

S.Y. Wang et al. beschreiben in CA 130:106409 (J. Am. Soc. Hortic. Sci., Bd. 123, Nr. 6, 1998, Seiten 987-991) die Anwendung von Riboflavin-Gemischen mit oberflächenaktiven Mitteln und Metallionen, um bestimmte Pilzinfektionen bei Erdbeerpflanzen zu verringern.

A.A. Aver'yanov et al. beschreiben in CA 134:111564 (Biochemistry Moscow), Band 65, Nr. 11, 2000, Seiten 1292-1298) die Kontrolle von bestimmten Reiserkrankungen mit Hilfe von Riboflavin und Roseoflavin.

D.D. Tzeng et al. beschreiben in CA 111:169241 (Mycologia, Bd. 81, Nr. 3, 1989, Seiten 404-412) die biozide

Wirkung von Methionin/Riboflavin-Mischungen gegen pflanzenpathogene Pilze und Bakterien, sowie mögliche Wirkmechanismen hierfür.

JP-A-51023299 offenbart die Herstellung von Roseoflavin und dessen stark antibakterielle Aktivität.

Die Suche nach neuen Chitinase-Inhibitoren, die sich zur Schädlingsbekämpfung eignen, ist daher von großem Interesse.

Die Erfindung betrifft daher die Verwendung von Flavinderivaten d er allgemeinen Formel (I)

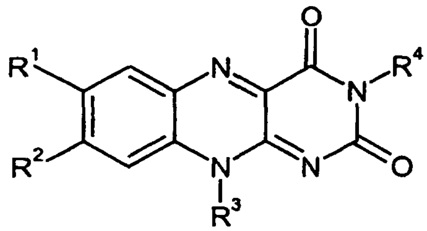

worin

$R^1$ für Wasserstoff, Trifluormethyl, Alkyl oder Alkoxy steht,

$R^2$ für Wasserstoff, Halogen, bevorzugt Chlor, Nitro, Alkyl, Alkoxy, Dialkylamino, bevorzugt Dimethylamino, $-CH_2COOH$ oder den Rest $NHCH_2CH(OH)CH(OH)CH(OH)CH_2OH$ steht,

$R^3$ für Wasserstoff, $CH_2CH_2CH(OH)CH(OH)CH_2OH$, $CH_2CH(OH)CH(OH)CH(OH)CH_2OH$, Phenyl, $-CH_2CHO$, $-CH_2CH_2OH$, $CH_2CH(OH)CH(OH)CH(OH)CH(OH)CH_3$ oder $CH_2CH(OH)CH(OH)CH(OH)CH_2OPO_3$ steht,

$R^4$ für Wasserstoff oder Alkyl steht

sowie von deren Stereoisomeren und Salzen zur Bekämpfung von Schädlingen ausgewählt aus Arthropoden, Nematoden und Chitin enthaltenden Pilzen. Erfindungsgemäß bevorzugt werden eingesetzt Verbindungen der Formel (I) worin

$R^1$ für Methyl

$R^2$ für Methyl, Dimethylamino, oder $NHCH_2CH(OH)CH(OH)CH(OH)CH_2OH$ steht

$R^3$ für $CH_2CH_2CH(OH)CH(OH)CH_2OH$ oder $CH_2CH(OH)CH(OH)CH(OH)CH_2OH$

$R^4$ für Wasserstoff steht

sowie deren Stereoisomere und Salze.

Die erfindungsgemäßen Verbindungen der allgemeinen Formel (I) können in verschiedenen stereoisomeren Formen auftreten, die sich entweder wie Bild und Spiegelbild (Enantiomere), oder die sich nicht wie Bild und Spiegelbild (Diastereomere) verhalten. Die Erfindung betrifft sowohl die Enantiomeren als auch die Diastereomeren sowie deren jeweilige Mischungen. Die Racemformen lassen sich ebenso wie die Diastereomeren in bekannter Weise in die stereoisomer einheitlichen Bestandteile trennen.

Weiterhin können bestimmte Verbindungen in tautomeren Formen vorliegen. Dies ist dem Fachmann bekannt, und derartige Verbindungen sind ebenfalls vom Umfang der Erfindung umfaßt.

Die erfindungsgemäßen Verbindungen können auch als Salze vorliegen. Im Rahmen der Erfindung sind physiologisch unbedenkliche Salze bevorzugt.

Physiologisch unbedenkliche Salze können Salze der erfindungsgemäßen Verbindungen mit anorganischen oder organischen Säuren sein. Bevorzugt werden Salze mit anorganischen Säuren wie beispielsweise Chlorwasserstoffsäure, Bromwasserstoffsäure, Phosphorsäure oder Schwefelsäure, oder Salze mit organischen Carbon- oder Sulfonsäuren wie beispielsweise Essigsäure, Propionsäure, Maleinsäure, Fumarsäure, Äpfelsäure, Zitronensäure, Weinsäure, Milchsäure, Benzoesäure, oder Methansulfonsäure, Ethansulfonsäure, Benzolsulfonsäure, Toluolsulfonsäure oder Naphthalindisulfonsäure.

Physiologisch unbedenkliche Salze können ebenso Salze der erfindungsgemäßen Verbindungen mit Basen sein, wie beispielsweise Metall- oder Ammoniumsalze. Bevorzugte Beispiele sind Alkalimetallsalze (z.B. Natrium- oder

Kaliumsalze), Erdalkalisalze (z.B. Magnesium- oder Calciumsalze), sowie Ammoniumsalze, die abgeleitet sind von Ammoniak oder organischen Aminen, wie beispielsweise Ethylamin, Di- bzw. Triethylamin, Ethyldiisopropylamin, Ethanolamin, Di- bzw. Triethanolamin, Dicyclohexylamin, Dimethylaminoethanol, Dibenzylamin, N-Methylmorpholin, Dihydroabietylamin, 1-Ephenamin, Methylpiperidin Arginin, Lysin, Ethylendiamin oder 2-Phenylethylamin.

Alkyl steht im Rahmen der Erfindung für einen geradkettigen oder verzweigten Alkylrest bevorzugt mit bis zu 6, besonders bevorzugt bis zu 4, ganz besonders bevorzugt bis zu 3 Kohlenstoffatomen. Beispielsweise seien genannt: Methyl, Ethyl, n-Propyl, Isopropyl, t-Butyl, n-Pentyl und n-Hexyl.

Alkoxy steht für einen geradkettigen oder verzweigten Alkoxyrest bevorzugt mit bis zu 6, besonders bevorzugt bis zu 4, ganz besonders bevorzugt bis zu 3 Kohlenstoffatomen. Beispielsweise seien genannt: Methoxy, Ethoxy, n-Propoxy, Isopmpoxy, tert.Butoxy, n-Pentoxy und n-Hexoxy.

Dialkylamino steht im Rahmen der Erfindung für eine Amino-Gruppe mit zwei gleichen oder verschiedenen geradkettigen oder verzweigten Alkylsubstituenten, die jeweils bevorzugt bis zu 6, besonders bevorzugt bis zu 4, ganz besonders bevorzugt bis zu 3 Kohlenstoffatome aufweisen. Beispielsweise seien genannt: *N,N* Dimethylamino, *N,N*-Diethylamino, *N*-Ethyl-*N*-methylamino, *N*-Methyl-*N*-n-propylamino, *N*-Isopropyl-*N*-n-propylamino, *N*-t-Butyl-*N*-methylamino, *N*-Ethyl-*N*-n-pentylamino und *N*-n-Hexyl-*N*-methylamino.

Die Verbindung Riboflavin ist bereits lange bekannt (siehe z. B. Merck-Index, 12. Auflage 1996, Eintrag Nr. 8367, S. 1410). Es handelt sich um die Verbindung der Formel (I) worin

$R^1$ und $R^2$ jeweils Methyl bedeuten

$R^3$ für den Rest der Formel

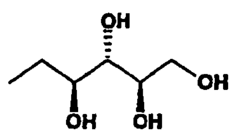

steht und

$R^4$ Wasserstoff bedeutet.

Die Chitinase inhibitorische Aktivität wurde bestimmt wie bereits beschrieben [Omura, S., Arai, N., Yamaguchi, Y., Masuma, R., Iwai, Y., Namikoshi, M., Turberg, A., Kölbl, H., Shiomi, K., J. Antibiot., 53, 603-608 (2000)]. Die $IC_{50}$ Werte von Riboflavin gegen Lucilia cuprina chitinase wurden bei 37°C mit 2,1 µM und bei 20 °C mit 0,38 µM bestimmt, aber es konnte keine Inhibition der Streptomyces griseus Chitinase (Sigma) oder der Vibrio alginolyticus Chitinase (Kyowa Medex) bei 100 µM (37°C) gemessen werden.

Aus der Literatur ist bekannt, das Riboflavin essentielle Aufgaben im Insekt haben muss. Die Zugabe von Riboflavin zu Futter (natürliche oder artifizielle Diäten) zeigten in mehreren Untersuchungen einen positiven Einfluss auf die Entwicklung von Larven und die Fertilität von adulten Insekten verschiedener Spezies. Es ist somit auch für den Fachmann überraschend das Riboflavin und Derivate des Riboflavins ein essentielles Enzym im Entwicklungsgeschehen von Arthropoden im µM Bereich hemmen und bei Behandlung von Insektenlarven teilweise zu einer Hemmung des Häutungsprozesses führen [Saksena, C. & S.L. Perti., Labdev J. Sci. Tech. 9-B, 126-131 (1971)].

Mehrere Autoren fanden, dass Riboflavin zwar in relativ hohen Konzentrationen in der Hämolymphe vorliegt, aber zu 100% an Proteine gebunden ist [Miller, S. G. & D. L. Silhacek, Insect Biochem. Molec. Biol. 22, 571-583 (1992)]. Der Riboflavin-Bindung wird allgemein eine Speicherfunktion zugesprochen - das essentielle Vitamin B2 soll so in ausreichender Menge auch in den Entwicklungsphasen zur Verfügung stehen, in denen keine Nahrung aufgenommen werden kann.

Findet man nun Riboflavin oder Flavin-Derivate, die die Kutikula durchqueren und die Epidermis erreichen oder die den Darm in ausreichender Konzentration penetrieren, können diese Derivate durch die Inhibition der Chitinasen den Abbau der Endocuticulaschicht hemmen und so zu einer Störung der Häutung führen. Die Konsequenz ist letztlich, dass der Arthropode eine neue Cuticula synthetisiert aber die alte Cuticula nicht oder nicht vollständig verlassen kann. Wachstum und Nahrungsaufnahme werden verhindert und der Arthropode stirbt letztlich.

Prinzipiell können Vertreter vom Typ der Verbindungen der Formel (I) Chitinasen aus Arthropoden, Nematoden und Pilzen hemmen. Sie eignen sich somit zum Einsatz als Arthropodizide, Nematizide und Fungizide.

Die Wirkstoffe eignen sich bei guter Pflanzenverträglichkeit und günstiger Warmblütertoxizität zur Bekämpfung von tierischen Schädlingen, insbesondere Insekten, Spinnentieren und Nematoden, die in der Landwirtschaft, in

Forsten, im Vorrats- und Materialschutz sowie auf dem Hygienesektor vorkommen. Sie können vorzugsweise als Pflanzenschutzmittel oder Tierarzneimittel für Nutz- und Hobbytiere sowie in der Stall- und Haushaltshygiene eingesetzt werden. Sie sind gegen normal sensible und resistente Arten sowie gegen alle oder einzelne Entwicklungsstadien wirksam. Zu den oben erwähnten Schädlingen gehören:

Aus der Ordnung der Isopoda z.B. Oniscus asellus, Armadillidium vulgare, Porcellio scaber.

Aus der Ordnung der Diplopoda z.B. Blaniulus guttulatus.

Aus der Ordnung der Chilopoda z.B. Geophilus carpophagus, Scutigera spp..

Aus der Ordnung der Symphyla z.B. Scutigerella immaculata.

Aus der Ordnung der Thysanura z.B. Lepisma saccharina.

Aus der Ordnung der Collembola z.B. Onychiurus armatus,

Aus der Ordnung der Orthoptera z.B. Acheta domesticus, Gryllotalpa spp., Locusta migratoria migratorioides, Melanoplus spp., Schistocerca gregaria.

Aus der Ordnung der Blattaria z.B. Blatta orientalis, Periplaneta americana, Leucophaea maderae, Blattella germanica.

Aus der Ordnung der Dermaptera z.B. Forficula auricularia.

Aus der Ordnung der Isoptera z.B. Reticulitermes spp..

Aus der Ordnung der Phthiraptera z.B. Pediculus humanus corporis, Haematopinus spp., Linognathus spp., Trichodectes spp., Damalina spp..

Aus der Ordnung der Thysanoptera z.B. Hercinothrips femoralis, Thrips tabaci, Thrips palmi, Frankliniella accidentalis.

Aus der Ordnung der Heteroptera z.B. Eurygaster spp., Dysdercus intermedius, Piesma quadrata, Cimex lectularius, Rhodnius prolixus, Triatoma spp.

Aus der Ordnung der Homoptera z.B. Aleurodes brassicae, Bemisia tabaci. Trialeurodes vaporariorum, Aphis gossypii, Brevicoryne brassicae, Cryptomyzus ribis, Aphis fabae, Aphis pomi, Eriosoma lanigerum, Hyalopterus arundinis, Phylloxera vastatrix, Pemphigus spp., Macrosiphum avenae, Myzus spp., Phorodon humuli, Rhopalosiphum padi, Empoasca spp., Euscelis bilobatus, Nephotettix cincticeps., Lecanium corni, Saissetia oleae, Laodelphax striatellus, Nilaparvata lugens, Aonidiella aurantii, Aspidiotus hederae, Pseudococcus spp., Psylla spp.

Aus der Ordnung der Lepidoptera z.B. Pectinophora gossypiella, Bupalus piniarius. Cheimatobia brumata, Lithocolletis blancardella, Hyponomeuta padella, Plutella xylostella, Malacosoma neustria, Euproctis chrysorrhoea, Lymantria spp., Bucculatrix thurbenella, Phyllocnistis citrella, Agrotis spp., Euxoa spp., Feltia., Earias insulana, Heliothis spp., Mamestra brassicae, Panolis flammea, Spodoptera Spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudoretella, Cacoecia podana, Capua reticulana, Choristoneura fumiferana, Clysia ambiguella, Homona magnanima, Tortrix viridana., Cnaphalocerus spp., Oulema oryzae.

Aus der Ordnung der Coleoptera z.B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis Anthonomus spp., Sitophilus spp., Otiorrhynchus sulcatus, Cosmopolites sordidus, Ceuthonrrhynchus assimili, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica, Lissorhoptrus oryzophilus.

Aus der Ordnung der Hymenoptera z.B. Diprion spp., Hoplocampa spp., Lasius spp., Monomorium pharaonis, Vespa spp.

Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hypobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa, Hylemyia spp., Liriomyza spp..

Aus der Ordnung der Siphonaptera z.B. Xenopsylla cheopis, Ceratophyllus spp..

Aus der Klasse der Arachnida z.B. Scorpio maurus, Latrodectus mactans, Acarus siro, Argas spp., Omithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp.. Bryobia praetiosa, Panonychus spp., Tetranychus spp., Hemitarsonemus spp., Brevipalpus spp..

Zu den pflanzenparasitären Nematoden gehören z.B. Pratylenchus spp., Radopholus similis, Ditylenchus dipsaci, Tylenchulus semipenetrans, Heterodera spp., Globodera spp., Meloidogyne spp., Aphelenchoides spp., Longidorus spp., Xiphinema spp., Trichodorus spp., Bursaphelenchus spp..

Die erfindungsgemäßen Verbindungen der Formel (I) zeichnen sich insbesondere durch ihre inhibierende Wirkung bei Dipteren-Endochitinasen sowie durch ihre Wirkung gegen larvale Entwicklungsstadien von Vertretern der

Ordnung Blattaria aus.

Die erfindungsgemäßen Verbindungen können gegebenenfalls in bestimmten Konzentrationen bzw. Aufwandmengen auch als Herbizide und Mikrobizide, beispielsweise als Fungizide, Antimykotika und Bakterizide verwendet werden. Sie lassen sich gegebenenfalls auch als Zwischen- oder Vorprodukte für die Synthese weiterer Wirkstoffe einsetzen.

Erfindungsgemäß können alle Pflanzen und Pflanzenteile behandelt werden. Unter Pflanzen werden hierbei alle Pflanzen und Pflanzenpopulationen verstanden, wie erwünschte und unerwünschte Wildpflanzen oder Kulturpflanzen (einschließlich natürlich vorkommender Kulturpflanzen). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Organe der Pflanzen, wie Sproß, Blatt, Blüte und Wurzel verstanden werden, wobei beispielhaft Blätter, Nadeln, Stengel, Stämme, Blüten, Fruchtkörper, Früchte und Samen sowie Wurzeln, Knollen und Rhizome aufgeführt werden. Zu den Pflanzenteilen gehört auch Erntegut sowie vegetatives und generatives Vermehrungsmaterial, beispielsweise Stecklinge, Knollen, Rhizome, Ableger und Samen.

Die erfindungsgemäße Behandlung der Pflanzen und Pflanzenteile mit den Wirkstoffen erfolgt direkt oder durch Einwirkung auf deren Umgebung, Lebensraum oder Lagerraum nach den üblichen Behandlungsmethoden, z. B. durch Tauchen, Sprühen, Verdampfen, Vernebeln, Streuen, Aufstreichen und bei Vermehrungsmaterial, insbesondere bei Samen, weiterhin durch ein- oder mehrschichtiges Umhüllen.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoffimprägnierte Natur- und synthetische Stoffe sowie Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:

z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykolether, Alkylsulfonate, Alkylsulfate, Arylsulfonate sowie Einweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gew.-% Wirkstoff, vorzugsweise zwischen 0,5 und 90 %.

Der erfindungsgemäße Wirkstoff kann in seinen handelsüblichen Formulierungen sowie in den aus diesen Formulierungen bereiteten Anwendungsformen in Mischung mit anderen Wirkstoffen, wie Insektiziden, Lockstoffen, Sterilantien, Bakteriziden, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u.a.

Besonders günstige Mischpartner sind z.B. die folgenden:

**Fungizide:**

Aldimorph, Ampropylfos, Ampropylfos-Kalium, Andoprim, Anilazin, Azaconazol, Azoxystrobin,

Benalaxyl, Benodanil, Benomyl, Benzamacril, Benzamacrylisobutyl, Bialaphos, Binapacryl, Biphenyl, Bitertanol, Blasticidin-S, Bromuconazol, Bupirimat, Buthiobat,

Calciumpolysulfid, Capsimycin, Captafol, Captan, Carbendazim, Carboxin, Carvon, Chinomethionat (Quinomethionat), Chlobenthiazon, Chlorfenazol, Chloroneb, Chloropicrin, Chlorothalonil, Chlozolinat, Clozylacon, Cufraneb, Cymoxanil, Cyproconazol, Cyprodinil, Cyprofuram,

Debacarb, Dichlorophen, Diclobutrazol, Diclofluanid, Diclomezin, Dicloran, Diethofencarb, Difenoconazol, Dimethirimol, Dimethomorph, Diniconazol, Diniconazol-M, Dinocap, Diphenylamin, Dipyrithione, Ditalimfos, Dithianon, Dodemorph, Dodine, Drazoxolon,

Ediphenphos, Epoxiconazol, Etaconazol, Ethirimol, Etridiazol,

Famoxadon, Fenapanil, Fenarimol, Fenbuconazol, Fenfuram, Fenitropan, Fenpiclonil, Fenpropidin, Fenpropimorph, Fentinacetat, Fentinhydroxyd, Ferbam, Ferimzon, Fluazinam, Flumetover, Fluoromid, Fluquinconazol, Flurprimidol, Flusilazol, Flusulfamid, Flutolanil, Flutriafol, Folpet, Fosetyl-Alminium, Fosetyl-Natrium, Fthalid, Fuberidazol, Furalaxyl, Furametpyr, Furcarbonil, Furconazol, Furconazolcis, Furmecyclox,

Guazatin,

Hexachlorobenzol, Hexaconazol, Hymexazol,

Imazalil, Imibenconazol, Iminoctadin, Iminoctadinealbesilat, Iminoctadinetciacetat, Iodocarb, Ipconazol, Iprobenfos (IBP), Iprodione, Irumamycin, Isoprothiolan, Isovaledione,

Kasugamycin, Kresoximmethyl, Kupfer-Zubereitungen, wie: Kupferhydroxid, Kupfernaphthenat, Kupferoxychlorid, Kupfersulfat, Kupferoxid, Oxin-Kupfer und Bordeaux-Mischung,

Mancopper, Mancozeb, Maneb, Meferimzone, Mepanipyrim, Mepronil, Metalaxyl, Metconazol, Methasulfocarb, Methfuroxam, Metiram, Metomeclam, Metsulfovax, Mildiomycin, Myclobutanil, Myclozolin,

Nickeldimethyldithiocarbamat, Nitrothalisopropyl, Nuarimol,

Ofurace, Oxadixyl, Oxamocarb, Oxolinicacid, Oxycarboxim, Oxyfenthiin,

Paclobutrazol, Pefurazoat, Penconazol, Pencycuron, Phosdiphen, Pimaricin, Piperalin, Polyoxin, Polyoxorim, Probenazol, Prochloraz, Procymidon, Propamocarb, Propanosine-Natrium, Propiconazol, Propineb, Pyrazophos, Pyrifenox, Pyrimethanil, Pyroquilon, Pyroxyfur,

Quinconazol, Quintozen (PCNB),

Schwefel und Schwefel-Zubereitungen,

Tebuconazol, Tecloftalam, Tecnazen, Tetcyclacis, Tetraconazol, Thiabendazol, Thicyofen, Thifluzamide, Thiophanatemethyl, Thiram, Tioxymid, Tolclofosmethyl, Tolylfluanid, Triadimefon, Triadimenol, Triazbutil, Triazoxid, Trichlamid, Tricyclazol, Tridemorph, Triflumizol, Triforin, Triticonazol,

Uniconazol,

Validamycin A, Vinclozolin, Viniconazol,

Zarilamid, Zineb, Ziram sowie

Dagger G,
OK-8705,
OK-8801,
α-(1,1-Dimethylethyl)-β-(2-phenoxyethyl)-1H-1,2,4-triazol-1-ethanol,
α-(2,4-Dichlorphenyl)-β-fluor-b-propyl-1H-1,2,4-triazol-1-ethanol,
α-(2,4-Dichlorphenyl)-β-methoxy-a-methyl-1H-1,2,4-triazol-1-ethanol,
α-(5 Methyl-1,3-dioxan-5-yl)-β-[[4-(trifluormethyl)-phenyl]-methylen]-1H-1,2,4-triazol-1-ethanol,
(5RS,6RS)-6-Hydroxy-2,2,7,7-tetramethyl-5-(1H-1,2,4-triazol-1-yl)-3-octanon,
(E)-a-(Methoxyimino)-N-methyl-2-phenoxy-phenylacetamid,
{2-Methyl-1-[[[1-(4-methylphenyl)-ethyl]-amino]-carbonyl]-propyl}-carbaminsäure-1-isopropylester
1-(2,4-Dichlorphenyl)-2-(1H-1,2,4-triazol-1-yl)-ethanon-O-(phenylmethyl)-oxim,
1-(2-Methyl-1-naphthalenyl)-1H pyrrol-2,5-dion,
1-(3,5-Dichlorphenyl)-3-(2-propenyl)-2,5-pyrrolidindion,
1-[(Diiodmethyl)-sulfonyl]-4-methyl-benzol,
1-[[2-(2,4-Dichlorphenyl)-1,3-dioxolan-2-yl]-methyl]-1H-imidazol,
1-[[2-(4-Chlorphenyl)-3-phenyloxiranyl]-methyl]-1H-1,2,4-triazol,
1-[1-[2-[(2,4-Dichlorphenyl)-methoxy]-phenyl]-ethenyl]-1H-imidazol,
1-Methyl-5-nonyl-2-(phenyhnethyl)-3-pyrrolidinol,
2',6'-Dibrom-2-methyl-4'-trifluormethoxy-4'-trifluor-methyl-1,3-thiazol-5-carboxanilid,
2,2-Dichlor-N-[1-(4-chlorphenyl)-ethyl]-1-ethyl-3-methyl-cyclopropancarboxamid,
2,6-Dichlor-5-(methylthio)-4-pyrimidinyl-thiocyanat,
2,6-Dichlor-N-(4-tritluormethylbenzyl)-benzamid,
2,6-Dichlor-N-[[4-(trifluomethyl)-phenyl]-methyl]-benzamid,
2-(2,3,3-Triiod-2-propenyl)-2H-tetrazol,
2-[(1-Methylethyl)-sulfonyl]-5-(trichlormethyl)-1,3,4-thiadiazol,
2-[[6-Deoxy-4-O-(4-O-methyl-β-D-glycopyranosyl)-a-D-glucopyranosyl]-amino]-4-methoxy-1H -pyrrolo[2,3-d] pyrimidin-5-carbonitril,
2-Aminobutan,
2-Brom-2-(brommethyl)-pentandinitril,
2-Chlor-N-(2,3-dihydro-1,1,3-trimethyl-1H-inden-4-yl)-3-pyridincarboxamid,
2-Chlor-N-(2,6-dimethylphenyl)-N-(isothiocyanatomethyl)-acetamid,
2-Phenylphenol(OPP),
3,4-Dichlor-1-[4-(difluormethoxy)-phenyl]-1H pyrrol-2,5-dion,
3,5-Dichlor-N [cyan[(1-methyl-2 propynyl)-oxy]-methyl]-benzamid,
3-(1,1-Dimethylpropyl-1-oxo-1H-inden-2-carbonitril,
3-[2-(4-Chlorphenyl)-5-ethoxy-3-isoxazolidinyl]-pyridin,
4-Chlor-2-cyan-N,N-dimethyl-5-(4-methylphenyl)-1H-imidazol-1-sulfonamid,
4-Methyl-tetrazolo[1,5-a]quinazolin-5(4H)-on,
8-(1,1-Dimethylethyl)-N-ethyl-N propyl-1,4-dioxaspiro[4.5]decan-2-methanamin,
8-Hydroxychinolinsulfat,
9H-Xanthen-9-carbonsäure-2-[(phenylamino)-carbonyl]-hydrazid,
bis-(1-Methylethyl)-3-methyl-4-[(3-methylbenzoyl)-oxy]-2,5-thiophendicarboxylat,
cis-1-(4-Chlorphenyl)-2-(1H-1,2,4-triazol-1-yl)-cycloheptanol,
cis-4-[3-[4-(1,1-Dimethylpropyl)-phenyl-2-methylpropyl]-2,6-dimethyl-morpholin-hydrochlorid,
Ethyl-[(4-chlorphenyl)-azo]-cyanoacetat,
Kaliumhydrogencarbonat,
Methantetrathiol-Natriumsalz,
Methyl-1-(2,3-dihydro-2,2-dimethyl-1H-inden-1-yl)-1H-imidazol-5-carboxylat,
Methyl-N-(2,6-dimethylphenyl)-N-(5-isoxazolylcarbonyl)-DL-alaninat,
Methyl-N-(chloracetyl)-N-(2,6-dimethylphenyl)-DL-alaninat,
N-(2,3-Dichlor-4-hydroxyphenyl)-1-methyl-cyclohexancarboxamid,
N-(2,6-Dimethylphenyl)-2-methoxy-N-(tetrahydro-2-oxo-3-furanyl)-acetamid,
N-(2,6-Dimethylphenyl)-2-methoxy-N-(tetrahydro-2-oxo-3-thienyl)-acetamid,
N-(2-Chlor-4-nitrophenyl)-4-methyl-3-nitro-benzolsulfonamid,
N-(4-Cyclohexylphenyl)-1,4,5,6-tetrahydro-2-pyrimidinamin,
N-(4-Hexylphenyl)-1,4,5,6-tetrahydro-2-pyrimidinamin,
N-(5-Chlor-2-methylphenyl)-2-methoxy-N-(2-oxo-3-oxazolidinyl)-acetamid,
N-(6-Methoxy)-3-pyridinyl)-cyclopropancarboxamid,

N-[2,2,2-Trichlor-1-[(chloracetyl)-amino]-ethyl]-benzamid,
N-[3-Chlor-4,5-bis-(2-propinyloxy)-phenyl]-N'-methoxy-methanimidamid,
N-Fonnyl-N-hydroxy-DL-alanin -Natriumsalz,
O,O-Diethyl-[2-(dipropylamino)-2-oxoethyl]-ethylphosphoramidothioat,
O-Methyl-S-phenyl-phenylpropylphosphoramidothioate,
S-Methyl-1,2,3-benzothiadiazol-7-carbothioat,
spiro[2H]-1-Benzopyran-2,1'(3'H)-isobenzofuran]-3'-on,

**Bakterizide:**

Bronopol, Dichlorophen, Nitrapyrin, Nickel-Dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen.

**Insektizide / Akarizide / Nematizide:**

Abamectin, Acephate, Acetamiprid, Acrinathrin, Alanycarb, Aldicarb, Aldoxycarb, Alphacypermethrin, Alphamethrin, Amitraz, Avermectin, AZ 60541, Azadirachtin, Azamethiphos, Azinphos A, Azinphos M, Azocyclotin,

Bacillus popilliae, Bacillus sphaericus, Bacillus subtilis, Bacillus thuringiensis, Baculoviren, Beauveria bassiana, Beauveria tenella, Bendiocarb, Benfuracarb, Bensultap, Benzoximate, Betacyfluthrin, Bifenazate, Bifenthrin, Bioethanomethrin, Biopermethrin, BPMC, Bromophos A, Bufencarb, Buprofezin, Butathiofos, Butocarboxim, Butylpyridaben,

Cadusafos, Carbaryl, Carbofuran, Carbophenothion, Carbosulfan, Cartap, Chloethocarb, Chlorethoxyfos, Chlorfenapyr, Chlorfenvinphos, Chlorfluazuron, Chlormephos, Chlorpyrifos, Chlorpyrifos M, Chlovaporthrin, Cis-Resmethrin, Cispermethrin, Clocythrin, Cloethocarb, Clofentezine, Cyanophos, Cycloprene, Cycloprothrin, Cyfluthrin, Cyhalothrin, Cyhexatin, Cypermethrin, Cyromazine,

Deltamethrin, Demeton M, Demeton S, Demeton-S-methyl, Diafenthiuron, Diazinon, Dichlorvos, Diflubenzuron, Dimethoat, Dimethylvinphos, Diofenolan, Disulfoton, Docusatsodium, Dofenapyn,

Eflusilanate, Emamectin, Empenthrin, Endosulfan, Entomopfthora spp., Eprinomectin, Esfenvalerate, Ethiofencarb, Ethion, Ethoprophos, Etofenprox, Etoxazole, Etrimfos,

Fenamiphos, Fenazaquin, Fenbutatin oxide, Fenitrothion, Fenothiocarb, Fenoxacrim, Fenoxycarb, Fenpropathrin, Fenpyrad, Fenpyrithrin, Fenpyroximate, Fenvalerate, Fipronil, Fluazinam, Fluazuron, Flubrocythrinate, Flucycloxuron, Flucythrinate, Flufenoxuron, Flutenzine, Fluvalinate, Fonophos, Fosmethilan, Fosthiazate, Fubfenprox, Furathiocarb,

Granuloseviren

Halofenozide, HCH, Heptenophos, Hexaflumuron, Hexythiazox, Hydroprene,

Imidacloprid, Isazofos, Isofenphos, Isoxathion, Ivermectin,

Kernpolyederviren

Lambdacyhalothrin, Lufenuron

Malathion, Mecarbam, Metaldehyd, Methamidophos, Metharhizium anisopliae, Metharhizium flavoviride, Methidathion, Methiocarb, Methomyl, Methoxyfenozide, Metolcarb, Metoxadiazone, Mevinphos, Milbemectin, Monocrotophos,

Naled, Nitenpyram, Nithiazine, Novaluron

Omethoat, Oxamyl, Oxydemethon M

Paecilomyces fumosoroseus, Parathion A, Parathion M, Permethrin, Phenthoat, Phorat, Phosalone, Phosmet, Phosphamidon, Phoxim, Pirimicarb, Pirimiphos A, Pirimiphos M, Profenofos, Promecarb, Propoxur, Prothiofos, Prothoat, Pymetrozine, Pyraclofos, Pyresmethrin, Pyrethrum, Pyridaben, Pyridathion, Pyrimidifen, Pyriproxyfen,

Quinalphos,

Ribavirin

Salithion, Sebufos, Selamectin, Silafluofen, Spinosad, Sulfotep, Sulprofos,

Taufluvalinate, Tebufenozide, Tebufenpyrad, Tebupirimiphos, Teflubenzuron, Tefluthrin, Temephos, Temivinphos, Terbufos, Tetrachlorvinphos, Thetacypermethrin, Thiamethoxam, Thiapronil, Thiatriphos, Thiocyclam hydrogen oxalate, Thiodicarb, Thiofanox, Thuringiensin, Tralocythrin, Tralomethrin, Triarathene, Triazamate, Triazophos, Triazuron, Trichlophenidine, Trichlorfon, Triflumuron, Trimethacarb,

Vamidothion, Vaniliprole, Verticillium lecanii

YI 5302

Zetacypermethrin, Zolaprofos

(1R-cis)-[5-(Phenylmethyl)-3-furanyl)-methyl-3-[(dihydro-2-oxo-3(2H)-furanyliden)-methyl]-2,2-dimethylcyclopropancarboxylat
(3-Phenoxyphenyl)-methyl-2,2,3,3-tetramethylcyclopropanecarboxylat
1-[(2-Chlor-5-thiazolyl)methyl]tetrahydro-3,5-dimethyl-N-nitro-1,3,5-triazin-2(1H)-imin
2-(2-Chlor-6-fluorphenyl)-4-[4-(1,1-dimethylethyl)phenyl]-4,5-dihydro-oxazol
2-(Acetlyoxy)-3-dodecyl-1,4-naphthalindion
2-Chlor-N-[[[4-(1-phenylethoxy)-phenyl]-amino]-carbonyl]-benzamid
2-Chlor-N-[[[4-(2,2-dichlor-1,1-difluorethoxy)-phenyl]-amino]-carbonyl]-benzamid 3-Methylphenyl-propylcarbamat
4-[4-(4-Ethoxyphenyl)-4-methylpentyl]-1-fluor-2-phenoxy-benzol
4-Chlor-2-(1,1-dimethylethyl)-5-[[2-(2,6-dimethyl-4-phenoxyphenoxy)ethyl]thio]-3 (2H)-pyridazinon
4-Chlor-2-(2-chlor-2-methylpropyl)-5-[(6-iod-3 pyridinyl)methoxy]-3(2H)-pyridazinon
4-Chlor-5-[(6-chlor-3 pyridinyl)methoxy]-2-(3,4-dichlorphenyl)-3(2H)-pyridazinon Bacillus thuringiensis strain EG-2348
Benzoesäure [2-benzoyl-1-(1,1-dimethylethyl)-hydrazid
Butansäure 2,2-dimethyl-3-(2,4-dichlorphenyl)-2-oxo-1-oxaspiro[4.5]dec-3-en-4-yl-ester
[3-[(6-Chlor-3-pyridinyl)methyl]-2-thiazolidinyliden]-cyanamid
Dihydro-2-(nitromethylen)-2H-1,3-thiazine-3(4H)-carboxaldehyd
Ethyl-[2-[[1,6-dihydro-6-oxo-1-(phenylmethyl)-4-pyridazinyl]oxy]ethyl]-carbamat
N-(3,4,4-Trifluor-1-oxo-3-butenyl)-glycin
N-(4-Chlorphenyl)-3-[4-(difluormethoxy)phenyl]-4,5-dihydro-4-phenyl-1H-pyrazol-1-carboxamid
N-[(2-Chlor-5-thiazolyl)methyl]-N-methyl-N''-nitro-guanidin
N-Methyl-N'-(1-methyl-2-propenyl)-1,2-hydrazindicarbothioamid
N-Methyl-N-2 propenyl-1,2-hydrazindicarbothioamid
O,O-Diethyl-[2-(dipropylamino)-2-oxoethyl]-ethylphosphoramidothioat

Auch eine Mischung mit anderen bekannten Wirkstoffen, wie Herbiziden oder mit Düngemitteln und Wachstumsregulatoren ist möglich.
Die erfindungsgemäßen Wirkstoffe können ferner beim Einsatz als Insektizide in ihren handelsüblichen Formu-lierungen sowie in den aus diesen Formulierungen bereiteten Anwendungs-formen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.
Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 95 Gew.-% Wirkstoff, vorzugsweise zwischen 0,0001 und 1 Gew.-% liegen.
Die Anwendung geschieht in einer den Anwendungsformen angepaßten üblichen Weise.
Bei der Anwendung gegen Hygiene- und Vorratsschädlinge zeichnet sich der Wirkstoff durch eine hervorragende Residualwirkung auf Holz und Ton sowie durch eine gute Alkalistabilität auf gekälkten Unterlagen aus.

Die erfindungsgemäßen Wirkstoffe wirken nicht nur gegen Pflanzen-, Hygiene- und Vorratsschädlinge, sondern auch auf dem veterinärmedizinischen Sektor gegen tierische Parasiten (Ektoparasiten) wie Schildzecken, Lederzecken, Räudemilben, Laufmilben, Fliegen (stechend und leckend), parasitierende Fliegenlarven, Läuse, Haarlinge, Federlinge und Flöhe. Zu diesen Parasiten gehören:

Aus der Ordnung der Anoplurida z.B. Haematopinus spp., Linognathus spp., Pediculus spp., Phtirus spp., Solenopotes spp..

Aus der Ordnung der Mallophagida und den Unterordnungen Amblycerina sowie Ischnocerina z.B. Trimenopon spp., Menopon spp., Trinoton spp., Bovicola spp., Werneckiella spp., Lepikentron spp., Damalina spp., Trichodectes spp.,

Aus der Ordnung Diptera und den Unterordnungen Nematocerina sowie Felicopa spp.. Brachycerina z.B. Aedes spp., Anopheles spp., Culex spp., Simulium spp., Eusimulium spp)., Phlebotomus spp., Lutzomyia spp., Culicoides spp., Chrysops spp., Hybomitra spp., Atylotus spp., Tabanus spp., Haematopota spp., Philipomyia spp., Braula spp., Musca spp., Hydrotaea spp., Stomoxvs spp., Haematobia spp., Morellia spp., Fannia spp., Glossina spp., Calliphora spp., Lucilia spp., Chrysomyia spp., Wohlfahrtia spp., Sarcophaga ssp., Oestrus spp., Hypoderma spp., Gasterophilus spp., Hippobosca spp., Lipoptena spp., Melophagus spp..

Aus der Ordnung der Siphonapterida z.B. Pulex spp., Ctenocephalides spp., Xenopsylla spp., Ceratophyllus spp..

Aus der Ordnung der Heteropterida z.B. Cimex spp., Triatoma spp., Rhodnius spp., Panstrongylus spp..

Aus der Ordnung der Blattarida z.B. Blatta orientalis, Periplaneta americana, Blattela germanica, Supella spp..

Aus der Unterklasse der Acaria (Acarida) und den Ordnungen der Meta- sowie Mesostigmata z.B. Argas spp., Ornithodorus spp., Otobius spp., Ixodes spp., Amblyomma spp., Boophilus spp., Dermacentor spp., Haemophysalis spp., Hyalomma spp., Rhipicephalus spp., Dermanyssus spp., Raillietia spp., Pneumonyssus spp., Sternostoma spp., Varroa spp..

Aus der Ordnung der Actinedida (Prostigmata) und Acaridida (Astigmata) z.B. Acarapis spp., Cheyletiella spp)., Ornithocheyletia spp., Myobia spp., Psorergates spp., Demodex spp., Trombicula spp., Listrophorus spp., Acarus spp., Tyrophagus spp., Caloglyphus spp., Hypodectes spp., Pterolichus spp., Psoroptes spp., Chorioptes spp., Otodectes spp., Sarcoptes spp., Notoedres spp., Knemidocoptes spp., Cytodites spp., Laminosioptes spp..

Die erfindungsgemäßen Wirkstoffe der Formel (I) eignen sich auch zur Bekämpfung von Arthropoden, die landwirtschaftliche Nutztiere, wie z.B. Rinder, Schafe, Ziegen, Pferde, Schweine, Esel, Kamele, Büffel, Kaninchen, Hühner, Puten, Enten, Gänse, Bienen, sonstige Haustiere wie z.B. Hunde, Katzen, Stubenvögel, Aquarienfische sowie sogenannte Versuchstiere, wie z.B. Hamster, Meerschweinchen, Ratten und Mäuse befallen. Durch die Bekämpfung dieser Arthropoden sollen Todesfälle und Leistungsminderungen (bei Fleisch, Milch, Wolle, Häuten, Eiern, Honig usw.) vermindert werden, so daß durch den Einsatz der erfindungsgemäßen Wirkstoffe eine wirtschaftlichere und einfachere Tierhaltung möglich ist.

Die Anwendung der erfindungsgemäßen Wirkstoffe geschieht im Veterinärsektor in bekannter Weise durch enterale Verabreichung in Form von beispielsweise Tabletten, Kapseln, Tränken, Drenchen, Granulaten, Pasten, Boli, des feed-through-Verfahrens, von Zäpfchen, durch parenterale Verabreichung, wie zum Beispiel durch Injektionen (intramuskulär, subcutan, intravenös, intraperitonal u.a.), Implantate, durch nasale Applikation, durch dermale Anwendung in Form beispielsweise des Tauchens oder Badens (Dippen), Sprühens (Spray), Aufgießens (Pour-on und Spot-on), des Waschens, des Einpuderns sowie mit Hilfe von wirkstoffhaltigen Formkörpern, wie Halsbändern, Qhrmarken, Schwanzmarken, Gliedmaßenbändern, Halftern, Markierungsvorrichtungen usw.

Bei der Anwendung für Vieh, Geflügel, Haustiere etc. kann man die Wirkstoffe der Formel (I) als Formulierungen (beispielsweise Pulver, Emulsionen, fließfähige Mittel), die die Wirkstoffe in einer Menge von 1 bis 80 Gew.% enthalten, direkt oder nach 100 bis 10 000-facher Verdünnung anwenden oder sie als chemisches Bad verwenden.

Außerdem wurde gefunden, daß die erfindungsgemäßen Verbindungen eine hohe insektizide Wirkung gegen Insekten zeigen, die technische Materialien zerstören.

Beispielhaft und vorzugsweise - ohne jedoch zu limitieren - seien die folgenden Insekten genannt:

Käfer wie
Hylotrupes bajulus, Chlorophorus pilosis, Anobium punctatum, Xestobium rufovillosum, Ptilinus pecticornis, Dendrobium pertinex, Ernobius mollis, Priobium carpini, Lyctus brunneus, Lyctus africanus, Lyctus planicollis, Lyctus linearis, Lyctuc pubescens, Trogoxylon aequale, Minthes rugicollis, Xyleborus spec. Tryiptodendron spec. Apate monachus, Bostrychus capucins, Heterobostrychus brunneus, Sinoxylon spec., Dinoderus minutus.

Hautflügler wie
Sirex juvencus, Urocerus gigas, Urocerus gigas taignus, Urocems augur.

Termiten wie
Kalotermes flavicollis, Cryptotermes brevis, Heterotermes indicola, Retieulitermes flavipes, Reticulitermes santonensis, Reticulitermes lucifugus, Mastotermes darwiniensis, Zootermopsis nevadensis, Coptotermes formosanus, Borstenschwänze wie Lepisma saccharina.

Unter technischen Materialien sind im vorliegenden Zusammenhang nicht-lebende Materialien zu verstehen, wie vorzugsweise Kunststoffe, Klebstoffe, Leime, Papiere und Kartone, Leder, Holz, Holzverarbeitungsprodukte und Anstrichmittel.

Ganz besonders bevorzugt handelt es sich bei dem vor Insektenbefall zu schützenden Material um Holz und Holzverarbeitungsprodukte.

Unter Holz und Holzverarbeitungsprodukten, welche durch das erfindungsgemäße Mittel bzw. dieses enthaltende Mischungen geschützt werden kann, ist beispielhaft zu verstehen:

Bauholz, Holzbalken, Eisenbahnschwellen, Brückenteile, Bootsstege, Holzfahrzeuge, Kisten, Paletten, Container, Telefonmasten, Holzverkleidungen, Holzfenster und -türen, Sperrholz, Spanplatten, Tischlerarbeiten oder Holzprodukte, die ganz allgemein beim Hausbau oder in der Bautischlerei Verwendung finden.

Die Wirkstoffe können als solche, in Form von Konzentraten oder allgemein üblichen Formulierungen wie Pulver, Granulate, Lösungen, Suspensionen, Emulsionen oder Pasten angewendet werden.

Die genannten Formulierungen können in an sich bekannter Weise hergestellt werden, z.B. durch Vermischen der Wirkstoffe mit mindestens einem Lösungs- bzw. Verdünnungsmittel, Emulgator, Dispergier- und/oder Binde- oder Fixiermittels, Wasser-Repellent, gegebenenfalls Sikkative und UV-Stabilisatoren und gegebenenfalls Farbstoffen und Pigmenten sowie weiteren Verarbeitungshilfsmitteln.

Die zum Schutz von Holz und Holzwerkstoffen verwendeten insektiziden Mittel oder Konzentrate enthalten den erfindungsgemäßen Wirkstoff in einer Konzentration von 0,0001 bis 95 Gew.-%, insbesondere 0,001 bis 60 Gew.%.

Die Menge der eingesetzten Mittel bzw. Konzentrate ist von der Art und dem Vorkommen der Insekten und von dem Medium abhängig. Die optimale Einsatzmenge kann bei der Anwendung jeweils durch Testreihen ermittelt werden. Im allgemeinen ist es jedoch ausreichend 0,0001 bis 20 Gew.-%, vorzugsweise 0,001 bis 10 Gew.-%, des Wirkstoffs, bezogen auf das zu schützende Material, einzusetzen.

Als Lösungs- und/oder Verdünnungsmittel dient ein organisch-chemisches Lösungsmittel oder Lösungsmittelgemisch und/oder ein öliges oder ölartiges schwer flüchtiges organisch-chemisches Lösungsmittel oder Lösungsmittelgemisch und/oder ein polares organisch-chemisches Lösungsmittel oder Lösungsmittelgemisch und/oder Wasser und gegebenenfalls einen Emulgator und/oder Netzmittel.

Als organisch-chemische Lösungsmittel werden vorzugsweise ölige oder ölartige Lösungsmittel mit einer Verdunstungszahl über 35 und einem Flammpunkt oberhalb 30°C, vorzugsweise oberhalb 45°C, eingesetzt. Als derartige schwerflüchtige, wasserunlösliche, ölige und ölartige Lösungsmittel werden entsprechende Mineralöle oder deren Aromatenfraktionen oder mineralölhaltige Lösungsmittelgemische, vorzugsweise Testbenzin, Petroleum und/oder Alkylbenzol verwendet.

Vorteilhaft gelangen Mineralöle mit einem Siedebereich von 170 bis 220°C, Testbenzin mit einem Siedebereich von 170 bis 220°C, Spindelöl mit einem Siedebereich von 250 bis 350°C, Petroleum bzw. Aromaten vom Siedebereich von 160 bis 280°C, Terpentinöl und dgl. zum Einsatz.

In einer bevorzugten Ausführungsform werden flüssige aliphatische Kohlenwasserstoffe mit einem Siedebereich von 180 bis 210°C oder hochsiedende Gemische von aromatischen und aliphatischen Kohlenwasserstoffen mit einem Siedebereich von 180 bis 220°C und/oder Spindeöl und/oder Monochlornaphthalin, vorzugsweise (Monochlornaphthalin, verwendet.

Die organischen schwerflüchtigen öligen oder ölartigen Lösungsmittel mit einer Verdunstungszahl über 35 und einem Flammpunkt oberhalb 30°C, vorzugsweise oberhalb 45°C, können teilweise durch leicht oder mittelflüchtige organisch-chemische Lösungsmittel ersetzt werden, mit der Maßgabe, daß das Lösungsmittelgemisch ebenfalls eine Verdunstungszahl über 35 und einen Flammpunkt oberhalb 30°C, vorzugsweise oberhalb 45°C, aufweist und daß das Insektizid-Fungizid-Gemisch in diesem Lösungsmittelgemisch löslich oder emulgierbar ist.

Nach einer bevorzugten Ausführungsform wird ein Teil des organisch-chemischen Lösungsmittel oder Lösungsmittelgemisches oder ein aliphatisches polares organisch-chemisches Lösungsmittel oder Lösungsmittelgemisch ersetzt. Vorzugsweise gelangen Hydroxyl- und/oder Ester- und/oder Ethergruppen enthaltende aliphatische organisch-chemische Lösungsmittel wie beispielsweise Glycolether, Ester oder dgl. zur Anwendung.

Als organisch-chemische Bindemittel werden im Rahmen der vorliegenden Erfindung die an sich bekannten wasserverdünnbaren und/oder in den eingesetzten organisch-chemischen Lösungsmitteln löslichen oder dispergier- bzw. emulgierbaren Kunstharze und/oder bindende trocknende Öle, insbesondere Bindemittel bestehend aus oder enthaltend ein Acrylathatz, ein Vinylharz, z.B. Polyvinylacetat, Polyesterharz, Polykondensations- oder Polyadditionsharz, Polyurethanharz, Alkydharz bzw. modifiziertes Alkydharz, Phenolharz, Kohlenwasserstoffharz wie Inden-Cumaronharz, Siliconharz, trocknende pflanzliche und/oder trocknende Öle und/oder physikalisch trocknende Bindemittel

auf der Basis eines Natur- und/oder Kunstharzes verwendet.

Das als Bindemittel verwendete Kunstharz kann in Form einer Emulsion, Dispersion oder Lösung, eingesetzt werden. Als Bindemittel können auch Bitumen oder bituminöse Substanzen bis zu 10 Gew.-%, verwendet werden. Zusätzlich können an sich bekannte Farbstoffe, Pigmente, wasserabweisende Mittel, Geruchskorrigentien und Inhibitoren bzw. Korrosionsschutzmittel und dgl. eingesetzt werden.

Bevorzugt ist gemäß der Erfindung als organisch-chemische Bindemittel mindestens ein Alkydharz bzw. modifiziertes Alkydharz und/oder ein trocknendes pflanzliches Öl im Mittel oder im Konzentrat enthalten. Bevorzugt werden gemäß der Erfindung Alkydharze mit einem Ölgehalt von mehr als 45 Gew.-%, vorzugsweise 50 bis 68 Gew.-%, verwendet.

Das erwähnte Bindemittel kann ganz oder teilweise durch ein Fixierungsmittel-(gemisch) oder ein Weichmacher(gemisch) ersetzt werden. Diese Zusätze sollen einer Verflüchtigung der Wirkstoffe sowie einer Kristallisation bzw. Ausfällem vorbeugen. Vorzugsweise ersetzen sie 0,01 bis 30 % des Bindemittels (bezogen auf 100 % des eingesetzten Bindemittels).

Die Weichmacher stammen aus den chemischen Klassen der Phthalsäureester wie Dibutyl-, Dioctyl- oder Benzylbutylphthalat, Phosphorsäureester wie Tributylphosphat, Adipinsäureester wie Di-(2-ethylhexyl)-adipat, Stearate wie Butylstearat oder Amylstearat, Oleate wie Butyloleat, Glycerinether oder höhermolekulare Glykolether, Glycerinester sowie p-Toluolsulfonsäureester.

Fixierungsmittel basieren chemisch auf Polyvinylalkylethern wie z.B. Polyvinylmethylether oder Ketonen wie Benzophenon, Ethylenbenzophenon.

Als Lösungs- bzw. Verdünnungsmittel kommt insbesondere auch Wasser in Frage, gegebenenfalls in Mischung mit einem oder mehreren der oben genannten organisch-chemischen Lösungs- bzw. Verdünnungsmittel, Emulgatoren und Dispergatoren.

Ein besonders effektiver Holzschutz wird durch großtechnische Imprägnierverfahren, z.B. Vakuum, Doppelvakunm oder Druckverfahren, erzielt.

Die anwendungsfertigen Mittel können gegebenenfalls noch weitere Insektizide und gegebenenfalls noch ein oder mehrere Fungizide enthalten.

Als zusätzliche Zumischpartner kommen vorzugsweise die in der WO 94/29 268 genannten Insektizide und Fungizide in Frage. Die in diesem Dokument genannten Verbindungen sind ausdrücklicher Bestandteil der vorliegenden Anmeldung.

Als ganz besonders bevorzugte Zumischpartner können Insektizide, wie Chlorpyriphos, Phoxim, Silafluofin, Alphamethrin, Cyfluthrin, Cypermethrin, Deltamethrin, Permethrin, Imidacloprid, NI-25, Flufenoxuron, Hexaflumuron, Transfluthrin, Thiacloprid, Methoxyphenoxid und Triflumuron, sowie Fungizide wie Epoxyconazole, Hexaconazole, Azaconazole, Propiconazole, Tebuconazole, Cyproconazole, Metconazole, Imazalil, Dichlorfluanid, Tolylfluanid, 3-Iod-2-propinylbutylcarbamat, N-Octylisothiazolin-3-on und 4,5-Dichlor-N-octylisothiazolin-3-on, sein.

Zugleich können die erfindungsgemäßen Verbindungen zum Schutz vor Bewuchs von Gegenständen, insbesondere von Schiffskörpern, Sieben, Netzen, Bauwerken, Kaianlagen und Signalanlagen, welche mit See- oder Brackwasser in Verbindung kommen, eingesetzt werden.

Bewuchs durch sessile Oligochaeten, wie Kalkröhrenwürmer sowie durch Muscheln und Arten der Gruppe Ledamorpha (Entenmuscheln), wie verschiedene Lepas- und Scalpellum-Arten, oder durch Arten der Gruppe Balanomorpha (Seepocken), wie Balanus- oder Pollicipes-Species, erhöht den Reibungswiderstand von Schiffen und führt in der Folge durch erhöhten Energieverbrauch und darüber hinaus durch häufige Trockendockaufenthalte zu einer deutlichen Steigerung der Betriebskosten.

Neben dem Bewuchs durch Algen, beispielsweise Ectocarpus sp. und Ceramium sp., kommt insbesondere dem Bewuchs durch sessile Entomostraken-Gruppen, welche unter dem Namen Cirripedia (Rankenflußkrebse) zusammengefaßt werden, besondere Bedeutung zu.

Es wurde nun überraschenderweise gefunden, daß die erfindungsgemäßen Verbindungen allein oder in Kombination mit anderen Wirkstoffen, eine hervorragende Antifouling (Antibewuchs)-Wirkung aufweisen.

Durch Einsatz von erfindungsgemäßen Verbindungen allein oder in Kombination mit anderen Wirkstoffen, kann auf den Einsatz von Schwermetallen wie z.B. in Bis-(trialkylzinn)-sulfiden, Tri-n-butyl-zinnlaurat, Tri-n-butyl-zinnchlorid, Kupfer(I)-oxid, Triethyl-zinnchlorid, Tri-n-butyl-(2-phenyl-4-chlor-phenoxy)-zinn, Tri-butylzinnoxid, Molybdändisulfid, Antimonoxid, polymerem Butyltitanat, Phenyl-(bis-pyridin)-wismutchlorid, Tri-n-butyl-zinnfluorid, Manganethylen-bisthio-carbamat, Zink-dimethyl-dithio-carbamat, Zink-ethylen-bisthio-carbamat, Zink- und Kupfersalze von 2-Pyridin-thiol-1-oxid, Bis-dimethyl-dithio-carbamoyl-zinkethylenbisthio-carbamat, Zinkoxid, Kupfer(I)-ethylen-bisdithio-carbamat, Kupferthiocyanat, Kupfer-naphthenat und Tri-butyl-zinnhalogeniden verzichtet werden oder die Konzentration dieser Verbindungen entscheidend reduziert werden.

Die anwendungsfertigen Antifoulingfarben können gegebenenfalls noch andere Wirkstoffe, vorzugsweise Algizide, Fungizide, Herbizide, Molluskizide bzw. andere Antifouling-Wirkstoffe enthalten.

Als Kombinationspartner für die erfindungsgemäßen Antifouling-Mittel eignen sich vorzugsweise:

Algizide wie
2-tert.-Butyl-amino-4-cyclo-propyl-amino-6-methyl-thio-1,3,5-triazin, Dichlorophen, Diuron, Endothal, Fentinacetat, Isoproturon, Methabenzthiazuron, Oxyfluorfen, Quinoclamine und Terbutryn;

Fungizide wie
Benzo[b]thiophencarbonsäure-cyclohexylamid-S,S-dioxid, Dichlofluanid, Fluorfolpet, 3-Iod-2-pro-pinyl-butylcarbamat, Tolylfluanid und Azole wie Azaconazole, Cyproconazole, Epoxyconazole, Hexaconazole, Metconazole, Propiconazole und Tebuconazole;

Molluskizide wie
Fentinacetat, Metaldehyd, Methiocarb, Niclosamid, Thiodicarb und Trimethacarb; oder herkömmliche Antifouling-Wirkstoffe wie

4,5-Di-chlor-2-octyl-4-isothiazolin-3-on, Di-iod-methyl-paratrylsulfon, 2-(N,N Dimethyl-thiocarbamoyl-thio)-5-nitro-thiazyl, Kalium-, Kupfer-, Natrium- und Zinksalze von 2-Pyridin-thiol-1-oxid, Pyridin-triphenylboran, Tetrabutyldistannoxan, 2,3,-5,6-Tetra-chlor-4-(methyl-sulfonyl)-pyridin, 2,4,5,6-Tetra-chloroisophthalonitril, Tetra-methyl-thiuram-disulfid und 2,4,6-Tri-chlor-phenylmaleinimid.

Die verwendeten Antifouling-Mittel enthalten die erfindungsgemäßen Wirkstoff der erfindungsgemäßen Verbindungen in einer Konzentration von 0,001 bis 50 Gew.-%, insbesondere von 0,01 bis 20 Gew.-%.

Die erfindungsgemäßen Antifouling-Mittel enthalten desweiteren die üblichen Bestandteile wie z.B. in Ungerer, Chem. Ind. 1985, 37, 730-732 und Williams, Antifouling Marine Coatings, Noyes, Park Ridge, 1973 beschrieben.

Antifouling-Anstrichmittel enthalten neben den algiziden, fungiziden, molluskiziden und erfindungsgemäßen insektiziden Wirkstoffen insbesondere Bindemittel.

Beispiele für anerkannte Bindemittel sind Polyvinylchlorid in einem Lösungsmittelsystem, chlorierter Kautschuk in einem Lösungsmittelsystem, Acrylharze in einem Lösungsmittelsystem insbesondere in einem wäßrigen System, Vinylchlorid/Vinylacetat-Copolymersysteme in Form wäßriger Dispersionen oder in Form von organischen Lösungsmittelsystemen, Butadien/Styrol/Acrylnitril-Kautschuke, trocknende Öle, wie Leinsamenöl, Harzester oder modifizierte Hartharze in Kombination mit Teer oder Bitumina, Asphalt sowie Epoxyverbindungen, geringe Mengen Chlorkautschuk, chloriertes Polypropylen und Vinylharze.

Gegebenenfalls enthalten Anstrichmittel auch anorganische Pigmente, organische Pigmente oder Farbstoffe, welche vorzugsweise in Seewasser unlöslich sind. Ferner können Anstrichmittel Materialien, wie Kolophonium enthalten, um eine gesteuerte Freisetzung der Wirkstoffe zu ermöglichen. Die Anstriche können ferner Weichmacher, die rheologischen Eigenschaften beeinflussende Modifizierungsmittel sowie andere herkömmliche Bestandteile enthalten. Auch in Self-Polishing-Antifouling-Systemen können die erfindungsgemäßen Verbindungen oder die oben genannten Mischungen eingearbeitet werden.

Die Wirkstoffe eignen sich auch zur Bekämpfung von tierischen Schädlingen, insbesondere von Insekten, Spinnentieren und Milben, die in geschlossenen Räumen, wie beispielsweise Wohnungen, Fabrikhallen, Büros, Fahrzeugkabinen u.ä. vorkommen. Sie können zur Bekämpfung dieser Schädlinge allein oder in Kombination mit anderen Wirk- und Hilfsstoffen in Haushaltsinsektizid-Produkten verwendet werden. Sie sind gegen sensible und resistente Arten sowie gegen alle Entwicklungsstadien wirksam. Zu diesen Schädlingen gehören:

Aus der Ordnung der Scorpionidea z.B. Buthus occitanus.

Aus der Ordnung der Acarina z.B. Argas persicus, Argas reflexus, Bryobia ssp., Dermanyssus gallinae, Glyciphagus domesticus, Ornithodorus moubat, Rhipicephalus sanguineus, Trombicula alfreddugesi, Neutrombicula autumnalis, Dermatophagoides pteronissimus, Dermatophagoides forinae,

Aus der Ordnung der Araneae z.B. Aviculariidae, Araneidae.

Aus der Ordnung der Opiliones z.B. Pseudoscorpiones chelifer, Pseudoscorpiones cheiridium, Opiliones phalangium,

Aus der Ordnung der Isopoda z.B. Oniscus asellus, Porcellio scaber,
Aus der Ordnung der Diplopoda z.B. Blaniulus guttulatus, Polydesmus spp
Aus der Ordnung der Chilopoda z.B. Geophilus spp,.

Aus der Ordnung der Zygentoma z.B. Ctenolepisma spp., Lepisma saccharina, Lepismodes inquilinus,

Aus der Ordnung der Blattaria z.B. Blatta orientalies, Blattella germanica, Blattella asahinai, Leucophaea maderae, Panchlora spp., Parcoblatta spp., Periplaneta, australasiae, Periplaneta americana. Periplaneta brunnea, Periplaneta fuliginosa, Supella longipalpa.

Aus der Ordnung der Saltatoria z.B. Acheta domesticus.
Aus der Ordnung der Dermaptera z.B. Forficula auricularia.
Aus der Ordnung der Isoptera z.B. Kalotermes spp., Reticulitermes spp.
Aus der Ordnung der Psocoptera z.B. Lepinatus spp., Liposcelis spp.

Aus der Ordnung der Coleptera z.B. Anthrenus spp., Attagenus spp., Dermestes spp., Latheticus oryzae, Necrobia spp., Ptinus spp., Rhizopertha dominica, Sitophilus granarius, Sitophilus oryzae, Sitophilus zeamais, Stegobium paniceum.

Aus der Ordnung der Diptera z.B. Aedes aegypti, Aedes albopictus, Aedes taeniorhynchus, Anopheles spp., Calliphora erythrocephala, Chrysozona pluvialis, Culex quinquefasciatus, Culex pipiens, Culex tarsalis. Drosophila spp., Fannia canicularis, Musca domestica, Phlebotomus spp., Sarcophaga carnaria, Simulium spp., Stomoxys calcitrans, Tipula paludosa.

Aus der Ordnung der Lepidoptera z.B. Achroia grisella, Galleria mellonella, Plodia interpunctella, Tinea cloacella, Tinea pellionella, Tineola bissenlliella,

Aus der Ordnung der Siphonaptera z.B. Ctenocephalides canis, Ctenocephalides felis, Pulex irritans, Tunga penetrans, Xenopsylla cheopis.

Aus der Ordnung der Hymenoptera z.B. Camponotus herculeanus, Lasius fuliginosus. Lasius niger, Lasius umbratus, Monomorium pharaonis, Paravesnula spp., Tetramorium caespitum.

Aus der Ordnung der Anoplura z.B. Pediculus humanus capitis, Pediculus humanus corporis, Phthirus pubis.

Aus der Ordnung der Heteroptera z.B. Cimex hemipterus, Cimex lectularius, Rhodinus prolixus, Triatoma infestans.

Die Anwendung im Bereich der Haushaltsinsektizide erfolgt allein oder in Kombination mit anderen geeigneten Wirkstoffen wie Phosphorsäureestern, Carbamaten, Pyrethroiden, Wachstumsregulatoren oder Wirkstoffen aus anderen bekannten Insektizidklassen.

Die Anwendung erfolgt in Aerosolen, drucklosen Sprühmitteln, z.B. Pump- und Zerstäubersprays, Nebelautomaten, Foggern, Schäumen, Gelen, Verdampferprodukten mit Verdampferplättchen aus Cellulose oder Kunststoff, Flüssigverdampfern, Gel- und Membranverdampfern, propellergetriebenen Verdampfern, energielosen bzw. passiven Verdampfungssystemen, Mottenpapieren, Mottensäckchen und Mottengelen, als Granulate oder Stäube, in Streuködern oder Köderstationen.

Vertreter der allgemeinen Formel (I) waren als Chitinaseinhibitoren wirksam und/oder hatten eine Insektizide Wirkung im biologischen Test.

**Tabelle 1:** Strukturbeispiele

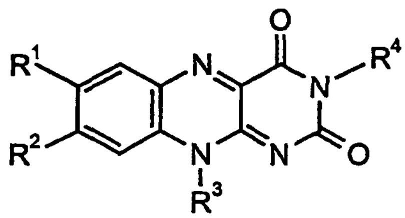

(I)

| Bsp. | R1 | R2 | R3 | R4 |
|---|---|---|---|---|
| 1 | $CH_3$ | $CH_3$ | $CH_2CH(OH)CH(OH)-CH(OH)CH_2OH$ | H |
| 2 | $CH_3$ | $CH_3$ | $CH_2CH(OH)CH(OH)-CH(OH)CH_2OH$ | $CH_3$ |
| 3 | $CH_3$ | $CH_2COOH$ | $CH_2CH(OH)CH(OH)-CH(OH)CH_2OH$ | H |
| 4 | $CH_3$ | $CH_3$ | $CH_2CH(OH)CH(OH)-CH(OH)CH_2OPO_3$ | H |
| 5 | $CH_3$ | $CH_3$ | $CH_2CHO$ | H |
| 6 | $CH_3$ | $N(CH_3)_2$ | $CH_2CH_2CH(OH)-CH(OH)CH_2OH$ | H |
| 7 | $CH_3$ | $-NHCH_2CH(OH)-CH(OH)CH(OH)CH_2OH$ | $CH_2CH(OH)CH(OH)-CH(OH)CH_2OH$ | H |
| 8 | H | Cl | $C_6H_6$ | H |
| 9 | H | $NO_2$ | H | H |
| 10 | H | Cl | $-CH_2CH_2OH$ | H |
| 11 | $CF_3$ | Cl | $CH_2CH(OH)CH(OH)-CH(OH)CH(OH)CH_3$ | H |

**Biologisches Beispiel 1**

Chitinasetest mit löslichen Extrakten aus *Lucilia cuprina* Puppen

Testtiere: Cytosol von *Lucilia cuprina*

Die in DMSO gelösten Substanzen werden auf 384-well Mikrotiterplatten in einer Konzentration von 2 mM zur Verfügung gestellt, wobei die ersten vier Spalten leer sind. Die Substanzen werden 1:40 mit 100 mM NPP pH 7.0 verdünnt (50 µM). Jeweils 5 µl dieser Verdünnungen werden auf eine 384-well Testplatte übertragen, beginnend mit Spalte 5. In die ersten beiden Spalten werden je als Negativkontrolle, in die dritte und vierte Spalte je 5 µl 5 x 10-6 M Allosamidin in 2.5 % DMSO in 100 mM NPP pH 7.0 5 µl 2.5 % DMSO in 100 mM NPP pH 7.0 als Positivkontrolle pipettiert.

Die Substanzen werden mit 2.5 µg cytosolischem Extrakt von späten Puppen von Lucilia cuprina, in 10 µl 100 mM Natriumphosphatpuffer pH 7.0 und 10 µl 100 uM Methylumbelliferyl (MUF-Triacetylchitotriose 20 Minuten bei Raumtemperatur inkubiert. Die Reaktion wird durch Zugabe von 25 µl 0.5 M Glycin-Puffer-2 M Natriumhydroxid, pH 10.4 gestoppt. Die Inhibition wird durch Messung der relativen Fluoreszenz bei 360 ± 40 nm Excitation und 460 ± 40 nm Emission bestimmt. Die Bewertung der zu testenden Substanzen wird im Vergleich zu einer Inhibition des Enzyms durch Allosamidin vorgenommen. Die Hemmung der MUF-Freisetzung ist ein Maß für die Wirksamkeit des Inhibitors. In der Nachtestung werden Konzentrationsreihen im Bereich von 100 nM bis 200 µM getestet und vor dem Abstoppen der Reaktion wird die MUF-Freisetzung alle 2 Minuten gemessen.

Als wirksam werden Verbindungen beurteilt, die bei maximaler Reaktionsgeschwindigkeit bei 100 µM mehr als 50% Inhibition der Chitinase zeigten.

Folgende Verbindungen zeigten beispielsweise mehr als 50% Inhibition der Endochitinase aus Lucilia cuprina bei 100 µM:

Bsp.: 1, 6, 7

| Bsp. | 200 µM | 100 µM | 10 µM |
|---|---|---|---|
| 1 | >>>50 | >>50 | >>50 |
| 6 | >>>50 | >>50 | >50 |
| 7 | >>>50 | >>50 | 50 |

**Biologisches Beispiel 2**

Injektionstest mit *Periplaneta* Larven

Testtiere: Dritte Larvenstadien von *Periplaneta americana*

Die Verbindungen werden in wässriger (0.1 N Essigsäure) Lösung angesetzt und entsprechend verdünnt. 1 µl der 20 bzw. 2 µg wurden hinter dem 3. Beinpaar mit einer 10 µl Hamiltonspritze flach unter die Bauchdecke der Schabenlarven injiziert. Als Kontrolle wurde Lösungsmittel (0.1 N Essigsäure), als Positivkontrolle 10 µg Allosamidin appliziert. Es wurden je zwanzig Schabenlarven pro Konzentration und Verbindung behandelt. Am Tag 1 wurde die Kontroll- und Initialmortalität, die im Wesentlichen durch artifizielle Effekte bei der Injektion hervorgerufen wurde bestimmt. Lag diese Anfangsmortalität unter 50% wurde der Versuch weitergeführt. Die Schabenlarven wurden auf Filterpapier in Standardpetrischalen (9.5 cm Ø) gesetzt und ein Viertel einer Oblate zugegeben. Über einen Zeitraum von 23 Tagen wurde die Häutungsrate der Kontrolltiere sowie der behandelten Schabenlarven beobachtet und die Larvenmortalität bzw. die Häutungshemmung nach 1, 5 und 23 Tagen notiert. Die Wirksamkeit wird nach folgender Formel errechnet.

$$\frac{100 \text{ x } (\text{\% Testmortalität - \% Kontrollmortalität})}{100 \text{ - \% Kontrollmortalität}}$$

Dabei werden solche Präparate als wirksam betrachtet, die bei der getesteten Konzentration eine Wirkung von mehr als 50 % zeigten.

Es zeigte beispielsweise Verbindung 1 mehr als 50% Mortalität gegen dritte Larvenstadien von Periplaneta americana bei 2 µg.

## Patentansprüche

**1.** Verwendung von Flavinderivaten der allgemeinen Formel (I)

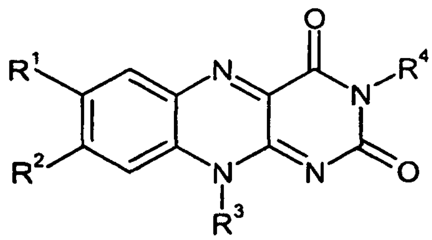

worin

$R^1$ für Wasserstoff, Trifluormethyl, Alkyl oder Alkoxy steht,

$R^2$ für Wasserstoff, Halogen, bevorzugt Chlor, Nitro, Alkyl, Alkoxy, Dialkylamino, bevorzugt Dimethylamino, $-CH_2COOH$ oder den Rest $NHCH_2CH(OH)CH(OH)CH(OH)CH_2OH$ steht,

$R^3$ für Wasserstoff, $CH_2CH_2CH(OH)CH(OH)CH_2OH$, $CH_2CH(OH)CH(OH)CH(OH)CH_2OH$, Phenyl, $-CH_2CHO$, $-CH_2CH_2OH$, $CH_2CH(OH)CH(OH)CH(OH)CH(OH)CH_3$ oder $CH_2CH(OH)CH(OH)CH(OH)CH_2OPO_3$ steht,

$R^4$ für Wasserstoff oder Alkyl steht

sowie von deren Stereoisomeren und Salzen als Chitinase-Inhibitoren zur Bekämpfung von Schädlingen ausgewählt aus Arthropoden, Nematoden und Chitin enthaltenden Pilzen.

**2.** Verwendung von Verbindungen der Formel (I) sowie von deren Stereoisomeren und Salzen gemäß Anspruch 1, **dadurch gekennzeichnet, dass**

$R^1$ für Methyl

$R^2$ für Methyl, Dimethylamino, oder $NHCH_2CH(OH)CH(OH)CH(OH)CH_2OH$

$R^3$ für $CH_2CH_2CH(OH)CH(OH)CH_2OH$ oder $CH_2CH(OH)CH(OH)CH(OH)CH_2OH$ und

$R^4$ für Wasserstoff steht.

**3.** Verwendung von Flavinderivaten der allgemeinen Formel (I), definiert wie in Anspruch 1 oder 2, sowie von deren Stereoisomeren und Salzen zur Bekämpfung von Schädlingen aus der Gruppe der Arthropoden und Nematoden.

**4.** Verwendung gemäß Anspruch 1 einer Kombination einer Verbindung der Formel (I) mit Schädlingsbekämpfungsmitteln aus der Klasse der Pyrethroide, Pyrazole, Neonicotinoide, Avermectine, Spinosyne oder anderen Wirkstoffklassen mit adultizidem bzw. nicht entwicklungsinhibitorischem Wirkpotential.

**5.** Verwendung von Verbindungen der Formel (I), definiert wie in Anspruch 1 oder 2, sowie von deren Steroisomeren und Salzen zur Herstellung von Mitteln zur Bekämpfung von Schädlingen aus der Gruppe d er Arthropoden und Nematoden an Tieren.

## Claims

**1.** The use of flavin derivatives of the formula (I)

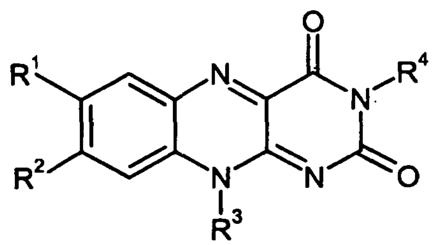

in which

$R^1$ is hydrogen, trifluoromethyl, alkyl or alkoxy,

$R^2$ is hydrogen, halogen, preferably chlorine, nitro, alkyl, alkoxy, dialkylamino, preferably dimethylamino, $-CH_2COOH$ or the radical $NHCH_2CH(OH)CH(OH)CH(OH)CH_2OH$,

$R^3$ is hydrogen, $CH_2CH_2CH(OH)CH(OH)CH_2OH$, $CH_2CH(OH)CH(OH)CH(OH)CH_2OH$, phenyl, $-CH_2CHO$, $-CH_2CH_2OH$, $CH_2CH(OH)CH(OH)CH(OH)CH(OH)CH_3$ or $CH_2CH(OH)CH(OH)CH(OH)CH_2OPO_3$,

$R^4$ is hydrogen or alkyl

and of their stereoisomers and salts as chitinase inhibitors for controlling pests selected from arthropods, nematodes and chitin-containing fungi.

**2.** The use of compounds of the formula (I) and of their stereoisomers and salts according to Claim 1, **characterized in that**

$R^1$ is methyl

$R^2$ is methyl, dimethylamino, or $NHCH_2CH(OH)CH(OH)CH(OH)CH_2OH$

$R^3$ is $CH_2CH_2CH(OH)CH(OH)CH_2OH$ or $CH_2CH(OH)CH(OH)CH(OH)CH_2OH$ and

$R^4$ is hydrogen.

**3.** The use of flavin derivatives of the formula (I), defined as in Claim 1 or 2, and of their stereoisomers and salts for controlling pests from the group of arthropods and nematodes.

**4.** The use according to Claim 1 of a combination of a compound of the formula (I) with pesticides from the class of the pyrethroids, pyrazoles, neonicotinoids, avermectins, spinosyns or other classes of active compounds with an active adulticidal or non-development-inhibitory potential.

**5.** The use of compounds of the formula (I), defined as in Claim 1 or 2, and of their stereoisomers and salts for the preparation of controlling compositions for pests from the group of arthropods and nematodes, in animals.

## Revendications

**1.** Utilisation de dérivés de la flavine de la formule générale (I) :

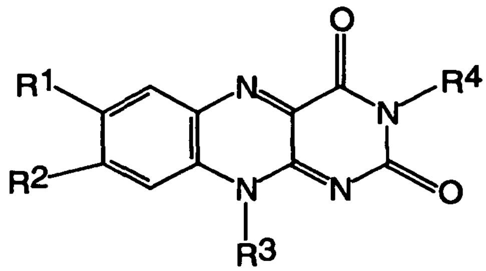

où

$R^1$ représente l'atome d'hydrogène, le radical trifluorométhyle, un radical alcoyle ou alcoxy,

$R^2$ représente l'atome d'hydrogène, un atome d'halogène, de préférence chlore, le radical nitro, un radical alcoyle, alcoxy, dialcoylamino, de préférence diméthylamino, $CH_2COOH$ ou le reste $NHCH_2CH$ (OH) CH (OH) CH (OH) $CH_2OH$,

$R^3$ représente l'atome d'hydrogène, le reste $CH_2CH_2CH(OH)CH(OH)CH_2OH$, $CH_2CH$ (OH) CH (OH) CH (OH) $CH_2OH$ , phényle , $CH_2CHO$, $CH_2CH_2OH$, $CH_2CH(OH)CH(OH)CH(OH)CH(OH)CH_3$, ou $CH_2CH$ (OH) CH (OH) CH (OH) $CH_2OPO_3$,

$R^4$ représente l'atome d'hydrogène ou un radical alcoyle,

ainsi que leurs stéréoisomères et sels comme inhibiteurs de chitinase pour lutter contre des parasites choisis parmi les arthropodes, les nématodes et les champignons contenant de la chitine.

**2.** Utilisation de composés de la formule (I) ainsi que de leurs stéréoisomères et sels selon la revendication 1, **caractérisée en ce que** :

$R^1$ représente le radical méthyle,
$R^2$ représente le radical méthyle, diméthylamino ou $NHCH_2CH(OH)CH(OH)CH(OH)CH_2OH$,
$R^3$ représente le reste $CH_2CH_2CH(OH)CH(OH)CH_2OH$ ou $CH_2CH(OH)CH(OH)CH(OH)CH_2OH$, et
$R^4$ représente l'atome d'hydrogène.

**3.** Utilisation de dérivés de la flavine de la formule générale (I), définis comme aux revendications 1 ou 2, ainsi que de leurs stéréoisomères et sels, pour lutter contre des parasites du groupe des arthropodes et des nématodes.

**4.** Utilisation suivant la revendication 1, d'une combinaison d'un composé de la formule (I) avec des agents de lutte contre les parasites de la classe des pyréthroïdes, pyrazoles, néonicotinoïdes, l'avermectine, le spinosyne ou d'autres classes d'agents actifs avec potentiel d'action adulticide et respectivement, non inhibiteurs du développement.

**5.** Utilisation de composés de la formule (I), définis comme aux revendications 1 ou 2, ainsi que de leurs stéréoisomères et sels, pour la préparation d'agents pour lutter contre des parasites du groupe des arthropodes et des nématodes sur les animaux.